Europäisches Patentamt

European Patent Office

Office européen des brevets

(19)

(11) Publication number: **0 117 034**
**B1**

(12) # EUROPEAN PATENT SPECIFICATION

(45) Date of publication of patent specification: **31.08.88**

(21) Application number: **84300150.4**

(22) Date of filing: **10.01.84**

(51) Int. Cl.⁴: **C 07 K 7/10,** A 61 K 37/02, A 23 K 1/16

(54) GRF analogs.

(30) Priority: **13.01.83 US 457862**
**26.04.83 US 488748**

(43) Date of publication of application:
**29.08.84 Bulletin 84/35**

(45) Publication of the grant of the patent:
**31.08.88 Bulletin 88/35**

(84) Designated Contracting States:
**AT BE CH DE FR GB IT LI LU NL SE**

(56) References cited:
**EP-A-0 121 764**
**EP-A-0 136 475**
**EP-A-0 137 689**
**EP-A-0 138 416**

**CHEMICAL ABSTRACTS, vol. 101, 1984, page
545, ref.nr. 38795e; Columbus, Ohio, US T.
KIMURA et al.: "Problems in the synthesis of
human parathyroid hormone."**

(73) Proprietor: **THE SALK INSTITUTE FOR
BIOLOGICAL STUDIES**
**10010 North Torrey Pines Road**
**La Jolla California 92037 (US)**

(72) Inventor: **Rivier, Jean-Edouard Frederic**
**9674 Blackgold Road**
**La Jolla California (US)**
Inventor: **Vale, Wylie Walker, Jr.**
**1643 Valdez**
**La Jolla California (US)**
Inventor: **Spiess, Joachim**
**271 Cerro Street**
**Encinitas California (US)**

(74) Representative: **Lawrence, Malcolm Graham
et al**
**Malcolm Lawrence & Co. 15th Floor Terminus
House Terminus Street**
**Harlow Essex CM20 1XD (GB)**

(56) References cited:
**NATURE, vol. 300, no. 5889, November 18,
1982; pages 276-278; Macmillan Journals Ltd.,
New York, US J. RIVIER et al.:
"Characterization of a growth hormone-
releasing factor from a human pancreatic islet
tumour."**

Courier Press, Leamington Spa, England.

# 0 117 034

**Description**

The present invention relates to a peptide having influence on the function of the pituitary gland in humans and other animals, particularly mammals. In particular, the present invention is directed to a peptide which promotes the release of growth hormone by the pituitary gland.

Physiologists have long recognized that the hypothalamus controls all the secretory function of the adenohypophysis with the hypothalamus producing special polypeptides which trigger the secretion of each pituitary hormone. An inhibitory factor has been characterized which inhibits the secretion of growth hormone (GH) and is termed somatostatin.

A corresponding hypothalamic releasing factor for pituitary GH has recently been isolated from an extract from a human pancreatic tumor, purified, characterized, synthesized and tested. The sequence of this peptide is as follows:

Tyr - Ala - Asp - Ala - Ile - Phe - Thr - Asn - Ser - Tyr - Arg - Lys - Val - Leu - Gly - Gln - Leu -
Ser - Ala - Arg - Lys - Leu - Leu - Gln - Asp - Ile - Met - Ser - Arg - Gln - Gln - Gly - Glu -
Ser - Asn - Gln - Glu - Arg - Gly - Ala - Arg - Ala - Arg - Leu.

The amidated form of this peptide is believed to be and is hereinafter referred to as hpGRF (for human pancreatic tumor GH releasing factor).

A polypeptide has now been isolated from rat hypothalamic extracts, purified, characterized, synthesized and tested which releases GH from cultured pituitary cells. The sequence of this 43-residue naturally occurring peptide is as follows:

H - His - Ala - Asp - Ala - Ile - Phe - Thr - Ser - Ser - Tyr - Arg - Arg - Ile - Leu - Gly - Gln - Leu -
Tyr - Ala - Arg - Lys - Leu - Leu - His - Glu - Ile - Met - Asn - Arg - Gln - Gln - Gly - Glu -
Arg - Asn - Gln - Glu - Gln - Arg - Ser - Arg - Phe - Asn - OH.

The peptide is hereinafter referred to as rhGRF (for rat hypothalamic GH releasing factor).

Certain analogs of hpGRF have now been synthesized and tested which are more potent than hpGRF itself, many of which analogs are shorter in length. Some of these analogs are based upon substitutions consistant with the formual of rhGRF whereas others are based upon other substitutions. Pharmaceutical compositions in accordance with the invention include these analogs of hpGRF, or a nontoxic salt thereof, dispersed in a pharmaceutically acceptable liquid or solid carrier. Such pharmaceutical compositions can be used in clinical medicine, both human and veterinary, for administration for therapeutic purposes, and also diagnostically. Moreover, they can be used to promote the growth of warm-blooded animals, including fowl, and in aquiculture for cold-blooded animals, e.g. fish, eels, etc.

The nomenclature used to define the peptide is that specified by Schroder & Lubke, "The Peptides", Academic Press (1965), wherein in accordance with conventional representation the amino group at the N-terminal appears to the left and the carboxyl group at the C-terminal to the right. By natural amino acid is meant one of common, naturally occurring amino acids found in proteins comprising Gly, Ala, Val, Leu, Ile, Ser, Thr, Lys, Arg, Asp, Asn, Glu, Gln Cys, Met, Phe, Tyr, Pro, Trp and His. Where the amino acid residue has isomeric forms, the L-form of the amino acid is being represented unless otherwise expressly indicated. For purposes of this application, rhGRF is considered to be an analog of hpGRF.

The invention provides synthetic hpGRF peptide analogs having the following formula:

H - $R_1$ - Ala - Asp - Ala - Ile - Phe - Thr - $R_8$ - Ser - $R_{10}$ - Arg - $R_{12}$ - $R_{13}$ - Leu - $R_{15}$ - Gln - Leu -
$R_{18}$ - Ala - Arg - Lys - Leu - Leu - $R_{24}$ - $R_{25}$ - Ile - $R_{27}$ - $R_{28}$ - Arg - Gln - Gln - Gly - Glu -
$R_{34}$ - Asn - Gln - Glu - $R_{38}$ - $R_{39}$ - $R_{40}$ - Arg - $R_{42}$ - $R_{43}$ - $R_{44}$ - Y

wherein $R_1$ is Tyr, Met, D-Tyr, Phe, D-Phe, Leu, D-His or His; $R_8$ is Ser or Asn; $R_{10}$ is Tyr, Phe or D-Tyr; $R_{12}$ is Arg or Lys; $R_{13}$ is Ile or Val; $R_{15}$ is Gly or D-Ala; $R_{18}$ is Tyr or Ser; $R_{24}$ is His or Gln; $R_{25}$ is Glu or Asp; $R_{27}$ is selected from the group consisting of the D- and L-isomers of Ala, Nle, Ile, Leu, Met and Val; $R_{28}$ is Ser, Asn or D-Ala; $R_{34}$ is Arg or Ser; $R_{38}$ is Gln or Arg; $R_{39}$ is Arg or Gly; $R_{40}$ is Ser or Ala; $R_{42}$ is Phe or Ala; $R_{43}$ is Asn or Arg; $R_{44}$ is a natural amino acid or des-$R_{44}$; and Y signifies the carboxyl moiety of the amino acid residue at the C-terminus and is the radical, —COOR, —CRO, —CONHNHR, —CON(R)(R') or —CH$_2$OR, with R and R' being lower alkyl, fluoro lower alkyl or hydrogen, provided however that when $R_1$ is Tyr, then $R_{27}$ is other than Met, $R_8$ is Ser, $R_{12}$ is Arg, $R_{13}$ is Ile, $R_{18}$ is Tyr, $R_{24}$ is His, $R_{25}$ is Glu, $R_{28}$ is Asn, $R_{34}$ is Arg, $R_{38}$ is Gln, $R_{39}$ is Arg, $R_{40}$ is Ser, $R_{42}$ is Phe or $R_{43}$ is Asn; provided further that any or all of residues 28 through 44 can be deleted to provide a biologically active fragment; or a nontoxic salt of the foregoing. Methyl, ethyl and propyl are the preferred lower alkyl groups. Fragments of foregoing peptides also have biological potency, and it is generally felt that the peptide should at least extend from the N-terminal through residue-27. When the peptide fragment extends only to residue 27 and 28, Y should be NH$_2$ or a substituted amide; whereas when the fragment extends to one of residues 29 thru 39, Y is preferably an amide or substituted amide.

The hpGRF analogs having one of the named substituents for Met in the 27-position exhibit substantially greater stability, particularly when exposed to oxidizing conditions; moreover, if the

2

substituent is a D-isomer, enzyme resistance may be enhanced. They remain fully biologically active, and certain analogs display surprisingly increased potency when tested *in vitro*. The substitution of His in the 1-position provides a surprisingly high potency. Furthermore, the substitution of a D-isomer amino acid, e.g. D-Tyr, retains a surprising amount of potency and may be quite valuable by rendering the peptide resistant to breakdown by certain enzymes.

The peptides are synthesized by a suitable method, such as by exclusively solid-phase techniques, by partial solid-phase techniques, by fragment condensation, by classical solution couplings, or by the employment of recently developed recombinant DNA techniques. For example, the techniques of exclusively solid-phase synthesis are set forth in the textbook "Solid-Phase Peptide Synthesis", Stewart & Young, Freeman & Co., San Francisco, 1969, and are exemplified by the disclosure of US—A—4,105,603, issued August 8, 1978 to Vale et al. The fragment condensation method of synthesis is exemplified in US—A—3,972,859 (August 3, 1976). Other available syntheses are exemplified by US—A—3,842,067 (October 15, 1974) and US—A—3,862,925 (January 28, 1975).

Synthesis by the use of recombinant DNA techniques, for purposes of this application, should be understood to include the suitable employment of a structural gene coding for the desired form of hpGRF analog or fragment thereof. The synthetic hpGRF peptide may be obtained by transforming a microorganism using an expression vector including a promoter and operator together with such structural gene and causing such transformed microorganism to express the hpGRF peptide. A non-human animal may also be used to produce the hpGRF peptide by gene-farming using such a structural gene and the general techniques set forth in U.S. Patent No. 4,276,282 issued June 30, 1981 or using microinjection of embryos as described in WO—A—83/01783 published 26 May 1983 and WO—A—82/04443 published 23 December 1982. The synthetic hpGRF peptide may also be produced directly in the animal for which accelerated growth is intended. The techniques described in the two WO publications may be employed for this purpose.

Common to chemical syntheses is the protection of the labile side chain groups of the various amino acid moieties with suitable protecting groups which will prevent a chemical reaction from occurring at that site until the group is ultimately removed. Usually also common is the protection of an alpha-amino group on an amino acid or a fragment while that entity reacts at the carboxyl group, followed by the selective removal of the alpha-amino protecting group to allow subsequent reaction to take place at that location. Accordingly, it is common that, as a step in the synthesis, an intermediate compound is produced which includes each of the amino acid residues located in its desired sequence in the peptide chain with side-chain protecting groups linked to the appropriate residues.

Peptides according to the present invention can be produced via intermediates of the formula:

$$X^1 - R_1 \, (X \, or \, X^2) - Ala - Asp(X^3) - Ala - Ile - Phe - Thr(X^4) - R_8(X^4 \, or \, X^5) - Ser(X^4) - R_{10}(X^2) - Arg(X^6) -$$
$$R_{12}(X^6 \, or \, X^7) - R_{13} - Leu - R_{15} - Gln(X^4) - Leu - R_{18}(X^2) - Ala - Arg(X^6) - Lys(X^7) - Leu - Leu -$$
$$R_{24}(X \, or \, X^5) - R_{25}(X^3) - Ile - R_{27} - R_{28}(X^4 \, or \, X^5) - Arg(X^6) - Gln(X^5) - Gln(X^5) - Gly - Glu(X^3) -$$
$$R_{34}(X^4 \, or \, X^6) - Asn(X^5) - Gln(X^5) - Glu(X^3) - R_{38}(X^5 \, or \, X^6) - R_{39}(X^6) - R_{40}(X^4) - Arg(X^6) - R_{42} -$$
$$R_{43}(X^5 \, or \, X^6) - R_{44}(X^8) - X^9$$

wherein: $X^1$ is either hydrogen or an α-amino protecting group. The α-amino protecting groups contemplated by $X^1$ are those well known to be useful in the art of step-wise synthesis of polypeptides. Among the classes of α-amino protecting groups which may be employed as $X^1$ are (1) aromatic urethan-type protecting groups, such as fluorenylmethyloxycarbonyl (FMOC), benzyloxycarbonyl(Z) and substituted Z, such as p - chlorobenzyloxycarbonyl, p - nitrobenzyloxycarbonyl, p - bromobenzyloxycarbonyl, and p - methoxybenzyloxycarbonyl; (2) aliphatic urethan protecting groups, such as t - butyloxycarbonyl (BOC), diisopropylmethyloxycarbonyl, isopropyloxycarbonyl, ethoxycarbonyl, allyloxycarbonyl; and (3) cycloalkyl urethan-type protecting groups, such as cyclopentyloxycarbonyl, adamantyloxycarbonyl, and cyclohexyloxycarbonyl. The preferred α-amino protecting group is BOC.

X is hydrogen or a protecting group for the imidazole nitrogen of His, such as Tos.

$X^2$ may be a suitable protecting group for the phenolic hydroxyl group of Tyr, such as tetrahydropyranyl, tert-butyl, trityl, Bzl, CBZ, 4Br-CBZ and 2,6-dichlorobenzyl (DCB). The preferred protecting group is 2,6-dichlorobenzyl. $X^2$ can be hydrogen which means that there is no side-chain protecting group on the amino acid residue in that position.

$X^3$ is hydrogen or a suitable ester-forming protecting group for the carboxyl group of Asp or Glu, such as benzyl (OBzl), 2,6-dichlorobenzyl, methyl and ethyl.

$X^4$ may be a suitable protecting group for the hydroxyl group of Thr or Ser, such as acetyl, benzoyl, tert-butyl, trityl, tetrahydropyranyl, Bzl, 2,6-dichlorobenzyl and CBZ. The preferred protecting group is Bzl. $X^4$ can be hydrogen, which means there is no protecting group on the hydroxyl group.

$X^5$ is hydrogen or a suitable protecting group for the side chain amido group or Asn or Gln. It is preferably xanthyl(Xan).

$X^6$ is a suitable protecting group for the guanidino group of Arg, such as nitro, Tos, CBZ, adamantyloxycarbonyl, and BOC, or is hydrogen.

$X^7$ is hydrogen or a suitable protecting group for the side chain amino group of Lys. Illustrative of

3

suitable side chain amino protecting groups are 2 - chlorobenzyloxycarbonyl (2-Cl-Z), Tos, t-amyloxycarbonyl and BOC.

$X^8$ is hydrogen or a suitable side-chain protecting group as generally specified above.

Met can optionally be protected by oxygen, but is preferably left unprotected.

The selection of a side chain amino protecting group is not critical except that generally one is chosen which is not removed during deprotection of the α-amino groups during the synthesis. However, for some amino acids, e.g. His, protection is not generally necessary after coupling is completed, and the protecting groups may be the same.

$X^9$ is a suitable protecting group for the C-terminal carboxyl group, such as the ester-forming group $X^3$, or is an anchoring bond used in solid-phase synthesis for linking to a solid resin support, or is des-$X^9$, in which case the residue at the C-terminal has a carboxyl moiety which is Y, as defined hereinbefore. When a solid resin support is used, it may be any of those known in the art, such as one having the formulae: —O—CH$_2$-resin support, —NH-benzhydrylamine (BHA) resin support or —NH-paramethylbenzhydrylamine (MBHA) resin support. When the unsubstituted amide is desired, use of BHA or MBHA resin is preferred, because cleavage directly gives the amide. In case the N-methyl amide is desired, it can be generated from an N-methyl BHA resin. Should other substituted amides be desired or should other groups than the free acid be desired at the C-terminus, it may be preferable to synthesize the peptide using classical methods as set forth in the Houben-Weyl text.

In the formula for the intermediate, at least one of the X-groups is a protecting group or $X^9$ includes resin support. Thus, the invention also provides a method for manufacturing a peptide of interest by (a) forming a peptide having at least one protective group and the formula (II):

$$X^1 - R_1 (X \text{ or } X^2) - Ala - Asp(X^3) - Ala - Ile - Phe - Thr(X^4) - R_8(X^4 \text{ or } X^5) - Ser(X^4) - Tyr(X^2) -$$
$$Arg(X^6) - R_{12}(X^6 \text{ or } X^7) - R_{13} - Leu - Gly - Gln(X^5) - Leu - R_{18}(X^2) - Ala - Arg(X^6) - Lys(X^7) -$$
$$Leu - Leu - R_{24}(X \text{ or } X^5) - R_{25}(X^3) - Ile - R_{27} - R_{28}(X^4 \text{ or } X^5) - Arg(X^6) - Gln(X^5) - Gln(X^5) -$$
$$Gly - Glu(X^3) - R_{34}(X^4 \text{ or } X^6) - Asn(X^5) - Gln(X^5) - Glu(X^3) - R_{38}(X^5 \text{ or } X^6) - R_{39}(X^6) - R_{40}(X^4) -$$
$$Arg(X^6) - R_{42} - R_{43}(X^5 \text{ or } X^6) - R_{44}(X^8) - X^9$$

wherein: X, $X^1$, $X^2$, $X^3$, $X^4$, $X^5$, $X^6$, $X^7$ and $X^8$ are each either hydrogen or a protective group and $X^9$ is either a protective group or an anchoring bond to resin support or des-$X^9$, in which case the residue at the C-terminal has a carboxy moiety which is Y; (b) splitting off the protective group or groups or anchoring bond from said peptide of the formula (II); and (c) if desired, converting a resulting peptide into a nontoxic salt thereof.

In selecting a particular side chain protecting group to be used in the synthesis of the peptides, the following rules are followed: (a) the protecting group must retain its protecting properties and not be split off under coupling conditions, (b) the protecting group should be stable to the reagent and, with the exception of Xan, should be stable under the reaction conditions selected for removing the α-amino protecting group at each step of the synthesis, and (c) the side chain protecting group must be removable, upon the completion of the synthesis containing the desired amino acid sequence, under reaction conditions that will not alter the peptide chain.

When peptides are not prepared using recombinant DNA technology, they are preferably prepared using solid phase synthesis, such as that generally described by Merrifield, *J. Am. Chem. Soc.*, 85, p. 2149 (1963), although other equivalent chemical syntheses known in the art can also be used as previously mentioned. Solid-phase synthesis is commenced from the C-terminal end of the peptide by coupling a protected α-amino acid to a suitable resin. Such a starting material can be prepared by attaching an α-amino-protected amino acid by an ester linkage to a chloromethylated resin or a hydroxymethyl resin, or by an amide bond to a BHA resin or MBHA resin. If a methyl, ethyl or propylamide is to be incorporated in the resulting polypeptide, a chloromethylated or hydroxymethyl resin is used, and cleavage is suitably effected by using the appropriate amine, e.g. ethylamine.

For example, in preparing the 40-residue peptide, Ala, protected by BOC, is coupled to the chloromethylated resin according to the procedure of Monahan and Gilon, *Biopolymer* 12, pp. 2513—19, 1973. Following the coupling of BOC-Ala to the resin support, the α-amino protecting group is removed, as by using trifluoroacetic acid (TFA) in methylene chloride, TFA alone or HCl in dioxane. The deprotection is carried out at a temperature between about 0°C and room temperature. Other standard cleaving reagents and conditions for removal of specific -amino protecting groups may be used as described in Schroder & Lubke, "The Peptides", 1 pp. 72—75 (Academic Press 1965).

After removal of the α-amino protecting group of Phe, the remaining α-amino- and side chain-protected amino acids are coupled step-wise in the desired order to obtain the intermediate compound defined hereinbefore, or as an alternative to adding each amino acid separately in the synthesis, some of them may be coupled to one another prior to addition to the solid phase reactor. The selection of an appropriate coupling reagent is within the skill of the art. Particularly suitable as a coupling reagent is N,N'-dicyclohexyl carbodiimide (DCCI).

The activating reagents used in the solid phase synthesis of the peptides are well known in the peptide art. Examples of suitable activating reagents are carbodiimides, such as N,N'-diisopropyl carbodiimide, N - ethyl - N' - (3 - dimethylaminopropyl)carbodiimide. Other activating reagents and their use in peptide coupling are described by Schroder & Lubke supra, in Chapter III and by Kapoor, *J. Phar. Sci.*, 59, pp. 1—27 (1970).

Each protected amino acid or amino acid sequence is introduced into the solid phase reactor in about a fourfold or more excess, and the coupling may be carried out in a medium of dimethyl-formamide(DMF):$CH_2Cl_2$ (1:1) or in DMF or $CH_2Cl_2$ alone. In cases where incomplete coupling occurs, the coupling procedure is repeated before removal of the α-amino protecting group prior to the coupling of the next amino acid. The success of the coupling reaction at each stage of the synthesis is monitored by the ninhydrin reaction, as described by E. Kaiser et al., *Anal. Biochem.* 34, 595 (1970). The coupling reactions can be performed automatically, as on a Beckman 990 automatic synthesizer, using a program such as that reported in Rivier et al. *Biopolymers*, 1978, 17, pp. 1927—1938.

After the desired amino acid sequence has been completed, the intermediate peptide can be removed from the resin support by treatment with a reagent, such as liquid hydrogen fluoride, which not only cleaves the peptide from the resin but also cleaves all remaining side chain protecting groups X, $X^2$, $X^3$, $X^4$, $X^5$, $X^6$, $X^7$ and $X^8$, the anchoring bond $X^9$ and the α-amino protecting group $X^1$, to obtain the peptide in the form of the free acid. If Met is present in the sequence, the BOC protecting group is preferably first removed using trifluoroacetic acid (TFA)/ethanedithiol prior to cleaving the peptide from the resin with HF to eliminate potential S-alkylation. When using hydrogen fluoride for cleaving, anisole and methylethyl sulfide are included in the reaction vessel for scavenging.

As one approach for preparing the synthetic peptides using recombinant DNA technology, a Met residue is preferably included as an additional residue at the N-terminus, and Met should not be present elsewhere in the peptide. Likewise, all of the residues employed in the peptide must be naturally occurring L-isomer amino acids or Gly. As a result, a preferred peptide intermediate composition is produced having the formula:

$$H - Met - R_1 - Ala - Asp - Ala - Ile - Phe - Thr - R_8 - Ser - Try - Arg - R_{12} - R_{13} - Leu - Gly - Gln -$$
$$Leu - R_{18} - Ala - Arg - Lys - Leu - Leu - R_{24} - R_{25} - Ile - R_{27} - R_{28} - Arg - Gln - Gln - Gly -$$
$$Glu - R_{34} - Asn - Gln - Glu - R_{38} - R_{39} - R_{40} - Arg - R_{42} - R_{43} - R_{44} - Y$$

wherein $R_1$ is Tyr, Leu or His; $R_8$ is Ser or Asn; $R_{12}$ is Arg or Lys; $R_{13}$ is Ile or Val; $R_{18}$ is Tyr or Ser; $R_{24}$ is His or Gln; $R_{25}$ is Glu or Asp; $R_{27}$ is Ala, Nle, Ile, Leu or Val; $R_{28}$ is Asn or Ser; $R_{34}$ is Arg or Ser; $R_{38}$ is Gln or Arg; $R_{39}$ is Arg or Gly; $R_{40}$ is Ser or Ala; $R_{42}$ is Phe or Ala; $R_{43}$ is Asn or Arg; $R_{44}$ is a natural amino acid or des-$R_{44}$, wherein Y represents the carboxyl moiety of the C-terminus residue and is either —COOH or $CONH_2$.

Once this peptide is expressed by a genetically modified microorganism and the intermediate composition is recovered and purified using known methods, the intermediate is treated with cyanogen bromide to cleave the Met residue at the N-terminus and produce the biologically active peptide. Although the formula set forth above indicates that the peptide will extend through position 43 or position 44, as indicated elsewhere herein, substantial biological activity is provided by peptides having as few as 27 to 29 residues, and such shorter fragments are considered to be generally equivalent for many purposes and to be included within the scope of the preferred intermediate that is provided by the invention which is intended for production by recombinant DNA technology.

The following Example sets forth the preferred method for synthesizing hpGRF analogs by the solid-phase technique. It will of course be appreciated that the synthesis of a correspondingly shorter peptide fragment is effected in the same manner by merely eliminating the requisite number of amino acids beginning at the C-terminal.

Example I

The synthesis of [Ile$^{27}$]-hpGRF(1—40)-$NH_2$ having the formula:

$$H - Tyr - Ala - Asp - Ala - Ile - Phe - Thr - Asn - Ser - Tyr - Arg - Lys - Val - Leu - Gly - Gln -$$
$$Leu - Ser - Ala - Arg - Lys - Leu - Leu - Gln - Asp - Ile - Ile - Ser - Arg - Gln - Gln - Gly - Glu -$$
$$Ser - Asn - Gln - Glu - Arg - Gly - Ala - NH_2$$

is conducted in a stepwise manner using a Beckman 990 peptide synthesizer on a MBHA hydrochloride resin, such as that available from Bachem, Inc., having a substitution range of about 0.1 to 0.5 mmoles/g resin. Coupling of BOC-Ala to the resin is performed by the general procedure set forth below in Schedules A and B which is used throughout the synthesis, and it results in the substitution of about 0.35 mmol Ala per gram of resin. All solvents that are used are carefully degassed by sparging with an inert gas, e.g. helium or nitrogen, to insure the absence of oxygen that might undesirably oxidize the sulfur of the Met residue.

After deblocking and neutralization, the peptide chain is built step-by-step on the resin. Deblocking, neutralization and addition of each amino acid is performed in general accordance with the procedure set forth in detail in Vale et al. US—A—4,292,313.

Deblocking is preferably carried out in accordance with Schedule A which follows:

Schedule A

| Reagent | Mixing time (min.) |
|---|---|
| 1. 60% TFA/2% ethanedithiol | 10 |
| 2. 60% TFA/2% ethanedithiol | 15 |
| 3. IPA/1% ethanedithiol | 0.5 |
| 4. Et$_3$N (10%) in CH$_2$Cl$_2$ | 0.5 |
| 5. MeOH | 0.5 |
| 6. Et$_3$N (10%) in CH$_2$Cl$_2$ | 0.5 |
| 7. MeOH (twice) | 0.5 |
| 8. CH$_2$Cl$_2$ (twice) | 0.5 |

The couplings are preferably carried out as set out in Schedule B which follows:

Schedule B

| Reagent | Mixing time (min.) |
|---|---|
| 9. DCCI | — |
| 10. Boc-amino acid | 50—90 |
| 11. MeOH (twice) | 0.5 |
| 12. CH$_2$Cl$_2$ (twice) | 0.5 |
| 13. Ac$_2$O (3M) in CH$_2$Cl$_2$ | 15.0 |
| 14. CH$_2$Cl$_2$ | 0.5 |
| 15. MeOH | 0.5 |
| 16. CH$_2$Cl$_2$ (twice) | 0.5 |

Briefly, one to two mmol of BOC-protected amino acid in methylene chloride is used per gram of resin, plus one equivalent of 1.0 molar DCCI in methylene chloride for two hours. When BOC-Arg(TOS) is being coupled, a mixture of 50% DMF and methylene chloride is used. Bzl ether is used as the hydroxyl side-chain protecting group for Ser and Thr. P-nitrophenyl ester (ONp) is used to activate the carboxyl end of Asn or Gln, and for example, BOC-Asn(ONp) is coupled overnight using one equivalent of HOBt in a 50% mixture of DMF and methylene chloride, in which case no DCC is added. The amido group of Asn or Gln is protected by Xan when DCC coupling is used instead of the active ester method. 2 - chlorobenzyloxycarbonyl (2Cl-Z) is used as the protecting group for the Lys side chain. Tos is used to protect the guanidino group of Arg, and the Glu or Asp carboxyl group is protected as the Bzl ester (OBzl). The phenolic hydroxyl group of Tyr is protected with 2,6-dichlorobenzyl (DCB). At the end of the synthesis, the following composition is obtained:

X$^1$ - Tyr(X$^2$) - Ala - Asp(X$^3$) - Ala - Ile - Phe - Thr(X$^4$) - Asn(X$^5$) - Ser(X$^4$) - Tyr(X$^2$) - Arg(X$^6$) - Lys(X$^7$) - Val - Leu - Gly - Gln(X$^5$) - Leu - Ser(X$^4$) - Ala - Arg(X$^6$) - Lys(X$^7$) - Leu - Leu - Gln(X$^5$) - Asp(X$^3$) - Ile - Ile - Ser(X$^4$) - Arg(X$^6$) - Gln(X$^5$) - Gln(X$^5$) - Gly - Glu(X$^3$) - Ser(X$^4$) - Asn(X$^5$) - Gln(X$^5$) - Glu(X$^3$) - Arg(X$^6$) - Gly - Ala - X$^8$

wherein X$^1$ is BOC, X$^2$ is DCB, X$^3$ is benzyl ester, X$^4$ is Bzl, X$^5$ is Xan, X$^6$ is Tos, X$^7$ is 2Cl-Za nd X$^8$ is —NH-resin support. Xan may have been partially or totally removed by TFA treatment used to deblock the α-amino protecting group.

After the final Tyr residue has been coupled to the resin, BOC is removed with 60% TFA in CH$_2$Cl$_2$. In order to cleave and deprotect the remaining protected peptide-resin, it is treated with 1.5 ml anisole, 0.5 ml methylethylsulfide and 15 ml hydrogen fluoride (HF) per gram of peptide-resin, at −20°C for one-half hour and at 0°C for one-half hour. After elimination of the HF under high vacuum, the resin-peptide remainder is washed alternately with dry diethyl ether and chloroform, and the peptide is then extracted with degassed 2N aqueous acetic acid and separated from the resin by filtration.

The cleaved and deprotected peptide is then dissolved in 0—5% acetic acid and subjected to purification which may include Sephadex® G-50 fine gel filtration.

The peptide is then further purified by preparative or semi-preparative HPLC as described in Rivier et al., *Peptides: Structure and Biological Function*, (1979) pp. 125—8 and Marki et al. *J. Am. Chem. Soc.* 103, 3178 (1981). In summary cartridges fitting Waters Associates prep LC-500 are packed with 15—20 $C_{18}$ Silica from Vydac (300A). A gradient of $CH_3CN$ in TEAP is generated by a low pressure Eldex gradient maker, as described in Rivier, J., *J. Liq. Chromatography 1*, 343—367 (1978). The chromatographic fractions are carefully monitored by HPLC, and only the fractions showing substantial purity are pooled. Desalting of the purified fractions, independently checked for purity, is achieved using a gradient of $CH_3CN$ in 0.1% TFA. The center cut is then lyophilized to yield the desired peptide, the purity of which is greater than 98%.

The synthesis is repeated using a chloromethylated resin to produce the same peptide in free acid form using procedures as generally described in Rivier, J, *J. Amer. Chem. Soc.*, 96, 2986—2992 (1974).

**Example II**

The synthesis of [Val$^{27}$, D-Ala$^{28}$]-hpGRF(1—4)-NH$_2$ having the formula:

H - Tyr - Ala - Asp - Ala - Ile - Phe - Thr - Asn - Ser - Tyr - Arg - Lys - Val - Leu - Gly - Gln - Leu - Ser - Ala - Arg - Lys - Leu - Leu - Gln - Asp - Ile - Val - D - Ala - Arg - Gln - Gln - Gly - Glu - Ser - Asn - Gln - Glu - Arg - Gly - Ala - NH$_2$

is conducted in a stepwise manner using a Beckman 990 Peptide Synthesizer on an MBHA resin in the manner described in Example I. The peptide is judged to be substantially pure using TLC and HPLC.

**Example III**

The synthesis of the hpGRF analog [D-Tyr$^1$]-hpGRF(1—32)-NH$_2$ having the formula:

H - D - Tyr - Ala - Asp - Ala - Ile - Phe - Thr - Asn - Ser - Tyr - Arg - Lys - Val - Leu - Gly - Gln - Leu - Ser - Ala - Arg - Lys - Leu - Leu - Gln - Asp - Ile - Met - Ser - Arg - Gln - Gln - Gly - NH$_2$

is conducted in a stepwise manner using a Beckman 990 Peptide Synthesizer on an MBHA resin, in the manner described in Example I. The peptide is judged to be substantially pure using TLC and HPLC. The optical rotation is measured on a photoelectric polarimeter as $[\alpha]_D^{22}=-61.4°\pm1$ (C=1, 1% acetic acid, uncorrected).

The synthesis is repeated using a chloromethylated resin to produce the same peptide in the free acid form as generally indicated hereinfore.

**Example IV**

The synthesis of the shortened hpGRF analog [Nle$^{27}$]-hpGRF(1—32)-NH$_2$ having the formula:

H - Tyr - Ala - Asp - Ala - Ile - Phe - Thr - Asn - Ser - Tyr - Arg - Lys - Val - Leu - Gly - Gln - Leu - Ser - Ala - Arg - Lys - Leu - Leu - Gln - Asp - Ile - Nle - Ser - Arg - Gln - Gln - Gly - NH$_2$

is conducted in a stepwise manner using a Beckman 990 Peptide Synthesizer on an MBHA resin in the manner described in Example I. This analog is judged to be substantially pure using TLC and HPLC. The optical rotation is measured on a photoelectric polarimeter as $[\alpha]_D^{22}=-57.8°\pm1$ (C=1, 1% acetic acid, uncorrected). The same general synthesis is used to make [Nle$^{27}$]-hpGRF (1—44)-NH$_2$.

**Example V**

The synthesis of [Nle$^{27}$]-hpGRF(1—27)-NH$_2$ having the formula:

H - Tyr - Ala - Asp - Ala - Ile - Phe - Thr - Asn - Ser - Tyr - Arg - Lys - Val - Leu - Gly - Gln - Leu - Ser - Ala - Arg - Lys - Leu - Leu - Gln - Asp - Ile - Nle - NH$_2$

is conducted in a stepwise manner using a Beckman 990 Peptide Synthesizer on an MBHA resin in the manner described in Example I. The peptide is judged to be substantially pure using TLC and HPLC.

**Example VI**

The synthesis of [Ile$^{27}$]-hpGRF(1—32)-OH having the formula:

H - Tyr - Ala - Asp - Ala - Ile - Phe - Thr - Asn - Ser - Tyr - Arg - Lys - Val - Leu - Gly - Gln - Leu - Ser - Ala - Arg - Lys - Leu - Leu - Gln - Asp - Ile - Ile - Ser - Arg - Gln - Gln - Gly - OH

is conducted in a stepwise manner using a Beckman 990 Peptide Synthesizer on a chloromethylated resin,

in the manner mentioned at the end of Example I. The peptide is judged to be substantially pure using TLC and HPLC. The optical rotation is measured on a photoelectric polarimeter as $[\alpha]_D^{22}=-61.7°\pm1$ (C=1, 1% acetic acid, uncorrected).

The synthesis is repeated using a MBHA resin to produce the same peptide in amidated form.

Example VII

The synthesis of [D-Met[27]]-hpGRF(1—32)-NH$_2$ having the formula:

H - Tyr - Ala - Asp - Ala - Ile - Phe - Thr - Asn - Ser - Tyr - Arg - Lys - Val - Leu - Gly - Gln - Leu - Ser - Ala - Arg - Lys - Leu - Leu - Gln - Asp - Ile - D - Met - Ser - Arg - Gln - Gln - Gly - NH$_2$

is conducted in a stepwise manner using a Beckman 990 Peptide Synthesizer on an MBHA resin in the manner described in Example I. The peptide is judged to be substantially pure using TLC and HPLC. The optical rotation is measured on a photoelectric polarimeter as $[\alpha]_D^{22}=-54.5°\pm1$ (C=1, 1% acetic acid, uncorrected).

Example VIII

The synthesis of [His[1]]-hpGRF(1—32)-NH$_2$ having the formula:

H - His - Ala - Asp - Ala - Ile - Phe - Thr - Asn - Ser - Tyr - Arg - Lys - Val - Leu - Gly - Gln - Leu - Ser - Ala - Arg - Lys - Leu - Leu - Gln - Asp - Ile - Met - Ser - Arg - Gln - Gln - Gly - NH$_2$

is conducted in a stepwise manner using a Beckman 990 Peptide Synthesizer on an MBHA resin in the manner described in Example I. The peptide is judged to be substantially pure using TLC and HPLC. The optical rotation is measured on a photoelectric polarimeter as $[\alpha]_D^{22}=-58.7°\pm1$ (C=1, 1% acetic acid, uncorrected).

The synthesis is repeated using Phe as the last residue to produce [Phe[1]]-hpGRF(1—32)-NH$_2$. The optical rotation is measured on a photoelectric polarimeter as $[\alpha]_D^{22}=-58.2°\pm1$ (C=1, 1% acetic acid, uncorrected).

Example IX

The synthesis of [Phe[10]]-hpGRF(1—32)-NH$_2$ having the formula:

H - Tyr - Ala - Asp - Ala - Ile - Phe - Thr - Asn - Ser - Phe - Arg - Lys - Val - Leu - Gly - Gln - Leu - Ser - Ala - Arg - Lys - Leu - Leu - Gln - Asp - Ile - Met - Ser - Arg - Gln - Gln - Gly - NH$_2$

is conducted in a stepwise manner using a Beckman 990 Peptide Synthesizer on an MBHA resin in the manner described in Example I. The peptide is judged to be substantially pure using TLC and HPLC. The optical rotation is measured on a photoelectric polarimeter as $[\alpha]_D^{22}=-59.5°\pm1$ (C=1, 1% acetic acid, uncorrected).

Example X

The synthesis of [Val[27]]-hpGRF(1—32)-OH having the formula:

H - Tyr - Ala - Asp - Ala - Ile - Phe - Thr - Asn - Ser - Tyr - Arg - Lys - Val - Leu - Gly - Gln - Leu - Ser - Ala - Arg - Lys - Leu - Leu - Gln - Asp - Ile - Val - Ser - Arg - Gln - Gln - Gly - OH

is conducted in a stepwise manner using a Beckman 990 Peptide Synthesizer on a chloromethylated resin in the manner mentioned at the end of Example I. The peptide is judged to be substantially pure using TLC and HPLC. The optical rotation is measured on a photoelectric polarimeter as $[\alpha]_D^{22}=-62.4°\pm1$ (C=1, 1% acetic acid, uncorrected).

Example XI

The synthesis of [His[1], Nle[27]]-hpGRF(1—32)-NH$_2$ having the formula:

H - His - Ala - Asp - Ala - Ile - Phe - Thr - Asn - Ser - Tyr - Arg - Lys - Val - Leu - Gly - Gln - Leu - Ser - Ala - Arg - Lys - Leu - Leu - Gln - Asp - Ile - Nle - Ser - Arg - Gln - Gln - Gly - NH$_2$

is conducted in a stepwise manner using a Beckman 990 Peptide Synthesizer on an MBHA resin in the manner described in Example I. The peptide is judged to be substantially pure using TLC and HPLC. The optical rotation is measured on a photoelectric polarimeter as $[\alpha]_D^{22}=-59.3°\pm1$ (C=1, 1% acetic acid, uncorrected).

Example XII

The synthesis of rhGRF(1—43)-OH having the formula:

H - His - Ala - Asp - Ala - Ile - Phe - Thr - Ser - Ser - Tyr - Arg - Arg - Ile - Leu - Gly - Gln - Leu -
Tyr - Ala - Arg - Lys - Leu - Leu - His - Glu - Ile - Met - Asn - Arg - Gln - Gln - Gly - Glu -
Arg - Asn - Gln - Glu - Gln - Arg - Ser - Arg - Phe - Asn - OH

is conducted in a stepwise manner using a Beckman 990 peptide synthesizer on a chloromethylated resin having a substitution range of about 0.1 to 0.5 mmoles/g resin in the manner indicated in Example VI. The peptide is judged to be substantially pure using TLC and HPLC. The optical rotation is measured on a photoelectric polarimeter as $[\alpha]_D^{22} = -50.8° \pm 1$ (C=1, 1% acetic acid, uncorrected).

The synthesis is repeated using an MBHA resin to produce rhGRF(1—43)-NH$_2$ using an initial procedure as generally described in Vale et al. U.S. Patent No. 4,292,313 to link Asn to the MBHA resin. The peptide is judged to be substantially pure using TLC and HPLC. The optical rotation is measured on a photoelectric polarimeter as $[\alpha]_D^{22} = -51.7° \pm 1$ (C=1, 1% acetic acid, uncorrected).

Example XIII

The synthesis of rhGRF(1—40)-NH$_2$ having the formula:

H - His - Ala - Asp - Ala - Ile - Phe - Thr - Ser - Ser - Tyr - Arg - Arg - Ile - Leu - Gly - Gln - Leu -
Tyr - Ala - Arg - Lys - Leu - Leu - His - Glu - Ile - Met - Asn - Arg - Gln - Gln - Gly - Glu -
Arg - Asn - Gln - Glu - Gln - Arg - Ser - NH$_2$

is conducted in a stepwise manner using a Beckman 990 Peptide Synthesizer on an MBHA resin as generally described in Example I. The peptide is judged to be substantially pure using TLC and HPLC. The optical rotation is measured on a photoelectric polarimeter as $[\alpha]_D^{22} = -56.5° \pm 1$ (C=1, 1% acetic acid, uncorrected).

Example XIV

The synthesis of a hpGRF analog, i.e. [Met$^1$, Leu$^{27}$]-rhGRF(1—43)-OH, having the formula:

H - Met - Ala - Asp - Ala - Ile - Phe - Thr - Ser - Ser - Tyr - Arg - Arg - Ile - Leu - Gly - Gln - Leu -
Tyr - Ala - Arg - Lys - Leu - Leu - His - Glu - Ile - Leu - Asn - Arg - Gln - Gln - Gly - Glu -
Arg - Asn - Gln - Glu - Gln - Arg - Ser - Arg - Phe - Asn - OH

is conducted in a stepwise manner using a Beckman 990 Peptide Synthesizer on a chloromethylated resin, in the manner generally described in Example XII. The peptide is judged to be substantially pure using TLC and HPLC.

Example XV

The synthesis of a hpGRF analog, i.e. [Nle$^{27}$]-rhGRF(1—32)-NH$_2$ having the formula:

H - His - Ala - Asp - Ala - Ile - Phe - Thr - Ser - Ser - Tyr - Arg - Arg - Ile - Leu - Gly - Gln - Leu -
Tyr - Ala - Arg - Lys - Leu - Leu - His - Glu - Ile - Nle - Asn - Arg - Gln - Gln - Gly - NH$_2$

is conducted in a stepwise manner using a Beckman 990 Peptide Synthesizer on an MBHA resin as in Example I. This analog is judged to be substantially pure using TLC and HPLC.

Example XVI

The synthesis of a hpGRF analog fragment i.e. [D-Tyr$^{10}$]-rhGRF(1—29)-NH$_2$ having the formula:

H - His - Ala - Asp - Ala - Ile - Phe - Thr - Ser - Ser - D - Tyr - Arg - Arg - Ile - Leu - Gly - Gln -
Leu - Tyr - Ala - Arg - Lys - Leu - Leu - His - Glu - Ile - Met - Asn - Arg - NH$_2$

is conducted in a stepwise manner using a Beckman 990 Peptide Synthesizer on an MBHA resin as in Example I. The peptide is judged to be substantially pure using TLC and HPLC.

The synthesis is repeated substituting D-Tyr for His at the N-terminus to produce [D-Tyr$^{1,10}$]-rhGRF(1—29)-NH$_2$.

Example XVII

The synthesis of a hpGRF analog fragment i.e. rhGRF(1—29)-NH$_2$, having the formula:

H - His - Ala - Asp - Ala - Ile - Phe - Thr - Ser - Ser - Tyr - Arg - Arg - Ile - Leu - Gly - Gln - Leu -
Tyr - Ala - Arg - Lys - Leu - Leu - His - Glu - Ile - Met - Asn - Arg - NH$_2$

9

is conducted in a stepwise manner using a Beckman 990 Peptide Synthesizer on an MBHA resin as in Example III. The peptide is judged to be substantially pure using TLC and HPLC.

Example XVIII

The synthesis of [Nle$^{27}$]-rhGRF(1—29)-NH$_2$ having the formula:

H - His - Ala - Asp - Ala - Ile - Phe - Thr - Ser - Ser - Tyr - Arg - Arg - Ile - Leu - Gly - Gln - Leu - Tyr - Ala - Arg - Lys - Leu - Leu - His - Glu - Ile - Nle - Asn - Arg - Gln - Gln - Gly - NH$_2$

is conducted in a stepwise manner using a Beckman 990 Peptide Synthesizer on an MBHA resin as in Example I. The peptide is judged to be substantially pure using TLC and HPLC. The optical rotation is measured on a photoelectric polarimeter as $[\alpha]_D^{22} = -52.6° \pm 1$ (C=1, 1% acetic acid, uncorrected).

Example XIX

The synthesis of a hpGRF analog i.e. [D-His$^1$, D-Tyr$^{10}$, D-Ala$^{15}$]-rhGRF(1—29)-NH$_2$ having the formula:

H - D - His - Ala - Asp - Ala - Ile - Phe - Thr - Ser - Ser - D - Tyr - Arg - Arg - Ile - Leu - D - Ala - Gln - Leu - Tyr - Ala - Arg - Lys - Leu - Leu - His - Glu - Ile - Met - Asn - Arg - NH$_2$

is conducted in a stepwise manner using a Beckman 990 Peptide Synthesizer on an MBHA resin as in Example I. The peptide is judged to be substantially pure using TLC and HPLC.

Example XX

The synthesis of a hpGRF analog i.e. [Tyr$^1$]-rhGRF(1—29)-NH$_2$ having the formula:

H - Tyr - Ala - Asp - Ala - Ile - Phe - Thr - Ser - Ser - Tyr - Arg - Arg - Ile - Leu - Gly - Gln - Leu - Tyr - Ala - Arg - Lys - Leu - Leu - His - Glu - Ile - Met - Asn - Arg - NH$_2$

is conducted in a stepwise manner using a Beckman 990 Peptide Synthesizer on an MBHA resin as in Example I. The peptide is judged to be substantially pure using TLC and HPLC.

Example XXI

The synthesis of rhGRF-[Val$^{44}$-NH$_2$] having the formula:

H - His - Ala - Asp - Ala - Ile - Phe - Thr - Ser - Ser - Tyr - Arg - Arg - Ile - Leu - Gly - Gln - Leu - Tyr - Ala - Arg - Lys - Leu - Leu - His - Glu - Ile - Met - Asn - Arg - Gln - Gln - Gly - Glu - Arg - Asn - Gln - Glu - Gln - Arg - Ser - Arg - Phe - Asn - Val - NH$_2$

is conducted in a stepwise manner using a Beckman 990 Peptide Synthesizer on an MBHA resin as in Example I. The peptide is judged to be substantially pure using TLC and HPLC.

In addition to the foregoing specifically enumerated analogs, the foregoing analogs are also synthesized using the same solid-phase synthesis techniques set forth hereinbefore:

[Ile$^{27}$]-hpGRF(1—29)-NH$_2$,
[Ile$^{27}$]-hpGRF(1—29)-OH,
[D-His$^1$]-hpGRF(1—32)-NH$_2$,
[D-Ala$^{27}$]-hpGRF(1—32)-NH$_2$,
[D-Tyr$^1$, Nle$^{27}$]-hpGRF(1—32)-NH$_2$,
[D-Tyr$^1$, Nle$^{27}$]-hpGRF(1—32)-OH,
[D-Phe$^1$, Val$^{27}$]-hpGRF(1—40)-OH,
[His$^1$, Phe$^{10}$]-hpGRF(1—32)-NH$_2$,
[D-Tyr$^1$, Nle$^{27}$]-hpGRF(1—40)-OH,
[D-Tyr$^1$, Nle$^{27}$]-hpGRF(1—40)-Arg-Ala-Arg-Leu-OH,
[Phe$^1$, Phe$^{10}$, Ile$^{27}$]-hpGRF(1—32)-NH$_2$,
[D-His$^1$, D-Ala$^{15}$, Nle$^{27}$]-hpGRF(1—32)-NH$_2$,
[D-Phe$^1$, Phe$^{10}$, d-Met$^{27}$]-hpGRF(1—40)-OH,
[Ile$^{27}$]-hpGRF(1—40)-Arg-Ala-Arg-Leu-NH$_2$,
[Ile$^{27}$]-hpGRF(1—40)-Arg-Ala-Arg-Leu-OH,
[D-His$^1$, D-Ala$^{15}$, D-Ala$^{28}$]-hpGRF(1—32)-NH$_2$,
[Phe$^1$, Leu$^{27}$, D-Ala$^{28}$]-hpGRF(1—40)-OH,
[His$^1$, D-Val$^{27}$, D-Ala$^{28}$]-hpGRF(1—32)-NH$_2$,
[D-Tyr$^1$, Phe$^{10}$, D-Ile$^{27}$]-hpGRF(1—39)-NH$_2$,
[Val$^{27}$]-hpGRF(1—40)-OH,
[Val$^{27}$]-hpGRF(1—40)-Arg-Ala-Arg-Leu-OH and
[D-Phe$^1$, D-Ala$^{15}$, D-Nle$^{27}$, D-Ala$^{28}$]-hpGRF(1—32)-NH$_2$.

10

**0 117 034**

The synthetic peptides prepared in various of the Examples are compared with either purified synthetic hpGRF(1—40)-OH or purified synthetic hpGRF(1—40)-Phe-Gln-NH$_2$ in *in vitro* assays to determine their effectiveness to promote the release of growth hormone. All these synthetic analogs have substantial potency in causing the release of GH.

*In vitro* assays are carried out using the synthetic hpGRF as a standard in side-by-side comparison with equimolar concentrations of the various analogs synthesized. Cultures are used which include cells of rat pituitary glands removed some four to five days previously. Cultures which are considered optimal for the secretion of growth hormone are used for the comparative testing, in the general manner described in Vale et al. *Endocrinology*, 91, 562—572 (1972). Incubation with the substance to be tested is carried out for 3 to 4 hours, and aliquots of the culture medium are removed and processed to measure their contents in immunoreactive GH(ir GH) by a well-characterized radioimmunoassay.

The results of this comparative testing using equimolar concentrations are shown in the following Table I:

TABLE I

| Peptide | *In vitro* Relative potency* |
|---|---|
| [Nle$^{27}$]-hpGRF(1—32)-NH$_2$ | 3.21(2.02—5.43) |
| [His$^1$]-hpGRF(1—32)-NH$_2$ | 2.15(1.12—4.41) |
| [Ile$^{27}$]-hpGRF(1—32)-OH | 0.20(0.11—0.35) |
| [D-Tyr$^1$]-hpGRF(1—32)-NH$_2$ | 0.74(0.47—1.14) |
| [D-Met$^{27}$]-hpGRF(1—32)-NH$_2$ | 0.22(0.13—0.36) |
| [Phe$^1$]-hpGRF(1—32)-NH$_2$ | 0.13(0.05—0.29) |
| [Phe$^{10}$]-hpGRF(1—32)-NH$_2$ | 0.69(0.26—2.63) |
| [Val$^{27}$]-hpGRF(1—32)-OH | 0.31(0.21—0.45) |
| [His$^1$, Nle$^{27}$]-hpGRF(1—32)-NH$_2$ | 3.22(2.12—5.33) |

*relative to hpGRF(1—40)-Phe-Gln-NH$_2$

*In vitro* testing of these synthetic peptides shows that the EC$_{50}$ varies from 20—100 picomolar and the lowest effective concentration to be 3—8 picomolar. The maximum effective concentration for hpGRF(1—40)NH$_2$ was 1 nanomolar.

The results of this comparative testing for equimolar concentrations of various of the hpGRF analogs based upon rhGRF are shown in Table II.

TABLE II

| Peptide | Comparison % |
|---|---|
| hpGRF(1—40)-OH (standard for this test) | 100% |
| rhGRF(1—43)-OH | 300% |
| rhGRF(1—29)-NH$_2$ | 1100% |
| [D-Tyr$^{10}$]-rhGRF(1—29)-NH$_2$ | 34% |
| [D-Tyr$^{1,10}$]-rhGRF(1—29)-NH$_2$ | 600% |
| [Tyr$^1$]-rhGRF(1—29)-NH$_2$ | 920% |
| [Nle$^{27}$]-rhGRF(1—32)-NH$_2$ | 1390% |
| [Leu$^1$, Nle$^{27}$]-rhGRF(1—29)-NH$_2$ | 480% |
| [D-His$^1$, D-Tyr$^{10}$, D-Ala$^{15}$]-rhGRF(1—29)-NH$_2$ | 29% |
| rhGRF-[Val$^{44}$-NH$_2$] | 670% |

11

*In vitro* testing of these synthetic peptides shows that the $EC_{50}$ varies from 20—100 picomolar and that the lowest effective concentration is 3—8 picomolar. The maximum effective concentration for rhGRF(1—43)-OH is 1 nanomolar.

In addition to the *in vitro* tests for secretion of growth hormone, *in vivo* experiments are also run by injecting the synthetic peptide through an indwelling catheter into freely running normal male rats. Animals are pretreated with FLA-63, a dopamine hydroxylase inhibitor that suppresses spontaneous GH secretion without affecting the response to exogenous GRF. Blood samples are taken through the same catheter immediately prior to and 5 and 20 minutes after injections; GH levels in blood are measured by radioimmunoassay. The results show that synthetic hpGRF analogs are powerful stimulators of the secretion of pituitary GH. Dosages between about 20 nanograms and about 25 micrograms per Kg of body weight are found to be effective.

Further testing shows that the other synthetic hpGRF analogs synthesized in the other Examples exhibit substantial potencies, and from this testing some general conclusions can be drawn:

1. C-terminal amidation does not appear to increase activity of GRF peptides of a chain length longer than 39 residues, but it does increase the potency of peptides shorter than 39 residues in length. For example,

[Ile$^{27}$]-hpGRF(1—32)-NH$_2$ is more potent than [Ile$^{27}$]-hpGRF(1—32)-OH;

[D-Tyr$^1$, Nle$^{27}$]-hpGRF(1—32)-NH$_2$ is more potent than [D-Tyr$^1$, Nle$^{27}$]-hpGRF(1—32)-OH; and

[Ile$^{27}$]-hpGRF(1—29)-NH$_2$ is more potent than [Ile$^{27}$]-hpGRF(1—29)-OH. However,

[Ile$^{27}$]-hpGRF(1—44)-NH$_2$ is equipotent with [Ile$^{27}$]-hpGRF(1—44)-OH.

2. In the series of amidated peptides, analogs of 32 residues are substantially as potent as the corresponding longer analogs; for example, [Nle$^{27}$]-hpGRF(1—32)-NH$_2$ has about the same potency as [Nle$^{27}$]-hpGRF(1—44)-NH$_2$.

3. In the series of peptides with free carboxy termini, corresponding analogs having greater than 39 residues are equipotent. For example, [Val$^{27}$]-hpGRF(1—40)-OH and [Val$^{27}$]-hpGRF(1—44)-OH are equipotent, as are [D-Tyr$^1$, Nle$^{27}$]-hpGRF(1—40)-OH and [D-Tyr$^1$, Nle$^{27}$]-hpGRF(1—44)-OH.

Synthetic hpGRF analogs should be useful for applications in which a physician wishes to elevate GH production. Stimulation of GH secretion by hpGRF analogs is of interest in patients with complete or relative GH deficiency caused by underproduction of endogenous GRF. Furthermore, it is possible that increased GH secretion and its attendant increase in growth could be obtained in humans or animals with normal GH levels. Furthermore, hpGRF administration should alter body fat content and modify other GH-dependent metabolic, immunologic and developmental processes. For example, hpGRF analogs may be useful as a means of stimulating anabolic processes in human beings under circumstances such as following the incurring of burns. In another example, hpGRF analogs may be administered to commercial animals, such as chickens, turkeys, pigs, goats, cattle and sheep, to accelerate growth and increase the ratio of protein to fat gained. They may be used in aquiculture for raising fish and other cold-blooded marine animals, e.g. sea turtles and eels, and amphibians. For administration to humans, synthetic hpGRF analogs should have a purity of at least about 93% and preferably at least 98%. This purity means the intended peptide constitutes the stated weight % of all like peptides and peptide fragments present.

For the administration of synthetic hpGRF analogs to commercial animals or other animals in order to promote growth and reduce fat content, a purity as low as about 5%, or even as low as about 0.001% may be acceptable. If produced by genetic engineering methods, the analogs may initially have very low purities.

Synthetic hpGRF analogs or the nontoxic salts thereof, combined with a pharmaceutically or veterinarily acceptable carrier to form a pharmaceutical composition, may be administered to animals, including humans, either intravenously, subcutaneously, intramuscularly, intranasally or orally. The administration may be employed by a physician to stimulate the release of GH where the host being treated requires such therapeutic treatment. The required dosage will vary with the particular condition being treated, with the severity of the condition and with the duration of desired treatment.

Such peptides are often administered in the form of pharmaceutically or veterinarily acceptable nontoxic salts, such as acid addition salts or metal complexes, e.g., with zinc, iron or the like (which are considered as salts for purposes of this application). Illustrative of such acid addition salts are hydrochloride, hydrobromide, sulphate, phosphate, maleate, acetate, citrate, benzoate, succinate, malate, ascorbate, tartrate and the like. If the active ingredient is to be orally administered in tablet form, the tablet may contain a binder, such as tragacanth, corn starch or gelatin; a disintegrating agent, such as alginic acid; and a lubricant, such as magnesium stearate. If administration in liquid form is desired, sweetening and/or flavoring may be used, and intravenous administration in isotonic saline, phosphate buffer solutions or the like may be effected.

The peptides should be administered to humans under the guidance of a physician, and pharmaceutical compositions will usually contain the peptide in conjunction with a conventional, pharmaceutically-acceptable carrier. Usually, the parenteral dosage will be from about 20 nanograms to about 25 micrograms of the peptide per kilogram of the body weight of the host.

**Claims for the Contracting States: BH, CH, DE, FR, GB, IT, LI, LU, NL, SE**

1. H - $R_1$ - Ala - Asp - Ala - Ile - Phe - Thr - $R_8$ - Ser - $R_{10}$ - Arg - $R_{12}$ - $R_{13}$ - Leu - $R_{15}$ - Gln - Leu - $R_{18}$ - Ala - Arg - Lys - Leu - Leu - $R_{24}$ - $R_{25}$ - Ile - $R_{27}$ - $R_{28}$ - Arg - Gln - Gln - Gly - Glu - $R_{34}$ - Asn - Gln - Glu - $R_{38}$ - $R_{39}$ - $R_{40}$ - Arg - $R_{42}$ - $R_{43}$ - $R_{44}$ - Y wherein $R_1$ is Tyr, Met, D-Tyr, Phe, D-Phe, Leu, D-His or His; $R_8$ is Ser or Asn; $R_{10}$ is Tyr, Phe or D-Tyr; $R_{12}$ is Arg or Lys; $R_{13}$ is Ile or Val; $R_{15}$ is Gly or D-Ala; $R_{18}$ is Tyr or Ser; $R_{24}$ is His or Gln; $R_{25}$ is Glu or Asp; $R_{27}$ is selected from the group consisting of the D- and L-isomers of Ala, Nle, Ile, Leu, Met and Val; $R_{28}$ is Ser, Asn or D-Ala; $R_{34}$ is Arg or Ser; $R_{38}$ is Gln or Arg; $R_{39}$ is Arg or Gly; $R_{40}$ is Ser or Ala; $R_{42}$ is Phe or Ala; $R_{43}$ is Asn or Arg; $R_{44}$ is a natural amino acid or des-$R_{44}$; and Y signifies the carboxyl moiety of the amino acid residue at the C-terminus and is the radical —COOR, —CRO, —CONHNHR, —CON(R)(R') or —CH$_2$OR, with R and R' being lower alkyl, fluoro lower alkyl or hydrogen, provided however that when $R_1$ is Tyr, then $R_{27}$ is other than Met, $R_8$ is Ser, $R_{12}$ is Arg, $R_{13}$ is Ile, $R_{18}$ is Tyr, $R_{24}$ is His, $R_{25}$ is Glu, $R_{28}$ is Asn, $R_{34}$ is Arg, $R_{38}$ is Gln, $R_{39}$ is Arg, $R_{40}$ is Ser, $R_{42}$ is Phe or $R_{43}$ is Asn; provided further that any or all of residues 28 through 44 can be deleted to provide a biologically active fragment; or a nontoxic salt of the foregoing.

2. A synthetic peptide having the formula of Claim 1 wherein $R_1$ is His.

3. A synthetic peptide having the formula or either Claim 1 or 2 wherein Y is CONH$_2$.

4. A synthetic peptide having the formula of any one of Claims 1—3 wherein $R_{27}$ is Nle.

5. A synthetic peptide having the formula of any one of Claims 1 to 4 wherein $R_{10}$ is Tyr, $R_{15}$ is Gly and $R_{28}$ is Ser.

6. A synthetic peptide having the formula of any one of Claims 1 to 4 wherein $R_{10}$ is Tyr, and $R_{15}$ is D-Ala and $R_{28}$ s D-Ala.

7. A synthetic peptide having the formula of Claim 1 wherein $R_1$ is D-Tyr and wherein $R_{27}$ is Nle, Val or Ile.

8. A synthetic peptide having the formula of either Claim 1 or 7 wherein $R_{15}$ is D-Ala and $R_{28}$ is D-Ala.

9. A synthetic peptide in accordance with Claim 1 having the sequence His - Ala - Asp - Ala - Ile - Phe - Thr - Ser - Ser - Tyr - Arg - Arg - Ile - Leu - Gly - Gln - Leu - Tyr - Ala - Arg - Lys - Leu - Leu - His - Glu - Ile - Met - Asn - Arg - Gln - Gln - Gly - Glu - Arg - Asn - Gln - Glu - Gln - Arg - Ser - Arg - Phe - Asn.

10. A synthetic peptide in accordance with any one of Claims 1—9 wherein residues 33 through 44 are deleted.

11. A non-toxic salt of a peptide as claimed in any one of Claims 2 to 10.

12. A composition for stimulating the release of GH in an animal which composition comprises a peptide as claimed in any one of Claims 1 to 10 or a nontoxic salt thereof, and a veterinarily or pharmaceutically acceptable liquid or solid carrier therefor.

13. A method of improving agriculture wherein release of GH in warm-blooded non-human animals is stimulated to promote increase in muscle mass by an effective amount of a peptide or salt as defined in Claim 1.

14. A method of improving agriculture wherein release of GH in cold-blooded animals is stimulated to accelerate the growth thereof by an effective amount of a peptide or salt as defined in Claim 1.

**Claims for the Contracting State: AT**

1. A process for the manufacture of compounds defined by the formula (I):

H - $R_1$ - Ala - Asp - Ala - Ile - Phe - Thr - $R_8$ - Ser - $R_{10}$ - Arg - $R_{12}$ - $R_{13}$ - Leu - $R_{15}$ - Gln - Leu — $R_{18}$ - Ala - Arg - Lys - Leu - Leu - $R_{24}$ - $R_{25}$ - Ile - $R_{27}$ - $R_{28}$ - Arg - Gln - Gln - Gly - Glu - $R_{34}$ - Asn - Gln - Glu - $R_{38}$ - $R_{39}$ - $R_{40}$ - Arg - $R_{42}$ - $R_{43}$ - $R_{44}$ - Y

wherein $R_1$ is Tyr, Met, D-Tyr, Phe, D-Phe, Leu, D-His or His; $R_8$ is Ser or Asn; $R_{10}$ is Tyr, Phe or D-Tyr; $R_{12}$ is Arg or Lys; $R_{13}$ is Ile or Val; $R_{15}$ is Gly or D-Ala; $R_{18}$ is Tyr or Ser; $R_{24}$ is His or Gln; $R_{25}$ is Glu or Asp; $R_{27}$ is selected from the group consisting of the D- and L-isomers of Ala, Nle, Ile, Leu, Met and Val; $R_{28}$ is Ser, Asn or D-Ala; $R_{34}$ is Arg or Ser; $R_{38}$ is Gln or Arg; $R_{39}$ is Arg or Gly; $R_{40}$ is Ser or Ala; $R_{42}$ is Phe or Ala; $R_{43}$ is Asn or Arg; $R_{44}$ is a natural amino acid or des-$R_{44}$; and Y signifies the carboxyl moiety of the amino acid residue at the C-terminus and is the radical —COOR, —CRO, —CONHNHR, —CON(R)(R') or —CH$_2$OR, with R and R' being lower alkyl, fluoro lower alkyl or hydrogen, provided however that when $R_1$ is Tyr, then $R_{27}$ is other than Met, $R_8$ is Ser, $R_{12}$ is Arg, $R_{13}$ is Ile, $R_{18}$ is Tyr, $R_{24}$ is His, $R_{25}$ is Glu, $R_{28}$ is Asn, $R_{34}$ is Arg, $R_{38}$ is Gln, $R_{39}$ is Arg, $R_{40}$ is Ser, $R_{42}$ is Phe or $R_{43}$ is Asn; provided further that any or all of residues 28 through 44 can be deleted to provide a biologically active fragment; or a nontoxic salt of the foregoing; comprising (a) forming a peptide having at least one protective group and the formula (II):

$X^1$ - $R_1$ (X or $X^2$) - Ala - Asp($X^3$) - Ala - Ile - Phe - Thr($X^4$) - $R_8$($X^4$ or $X^5$) - Ser($X^4$) - $R_{10}$($X^2$) - Arg($X^6$) - $R_{12}$($X^6$ or $X^7$) - $R_{13}$ - Leu - $R_{15}$ - Gln($X^5$) - Leu - $R_{18}$($X^2$) - Ala - Arg($X^6$) - Lys($X^7$) - Leu - Leu - $R_{24}$(X or $X^5$) - $R_{25}$($X^3$) - Ile - $R_{27}$ - $R_{28}$($X^4$ or $X^5$) - Arg($X^6$) - Gln($X^5$) - Gln($X^5$) - Gly - Glu($X^3$) - $R_{34}$($X^4$ or $X^6$) - Asn($X^5$) - Gln($X^5$) - Glu($X^3$) - $R_{38}$($X^5$ or $X^6$) - $R_{39}$($X^6$) - $R_{40}$($X^4$) - Arg($X^6$) - $R_{42}$ - $R_{43}$($X^5$ or $X^6$) - $R_{44}$($X^8$) - $X^9$

wherein: X, $X^1$, $X^2$, $X^3$, $X^4$, $X^5$, $X^6$, $X^7$ and $X^8$ are each either hydrogen or a protective group and $X^9$ is either a protective group or an anchoring bond to resin support or des-$X^9$, in which case the residue at the C-terminal has a carboxy moiety which is Y; (b) splitting off the protective group or groups or anchoring bond from said peptide of the formula (II); and (c) if desired, converting a resulting peptide into a nontoxic salt thereof.

2. A process in accordance with Claim 1 wherein $X^1$ is hydrogen or an α-amino protecting group; X is hydrogen or a protecting group for the imidazole nitrogen of His; $X^2$ is hydrogen or a protecting group for the phenolic hydroxyl group of Tyr; $X^3$ is hydrogen or a protecting group for the carboxyl group of Asp or Glu; $X^4$ is hydrogen or a protecting group for the alcoholic hydroxyl group of Ser and Thr; $X^5$ is hydrogen or a protecting group for the amido group of Asn or Gln; $X^6$ is hydrogen or a protecting group for the guanidino group of Arg; $X^7$ is hydrogen or a protecting group for the side chain amino group of Lys.

3. A process in accordance with Claim 2 wherein X is hydrogen or Tos, $X^1$ is BOC, $X^2$ is DCB, $X^3$ is OBzl, $X^4$ is Bzl, $X^5$ is Xan, $X^6$ is Tos, $X^7$ is 2 - chlorobenzyloxycarbonyl and $X^8$ is —NH-resin support.

4. A process in accordance with any one of Claims 1—3 wherein $R_1$ is His.

5. A process in accordance with any one of Claims 1—4 wherein Y is $CONH_2$.

6. A process in accordance with any one of Claims 1—5 wherein $R_{27}$ is Nle.

7. A process in accordance with any one of Claims 1 to 6 wherein $R_{10}$ is Tyr, $R_{15}$ is Gly and $R_{28}$ is Ser.

8. A process in accordance with any one of Claims 1 to 6 wherein $R_{10}$ is Tyr, $R_{15}$ is D-Ala and $R_{28}$ is D-Ala.

9. A process in accordance with any one of Claims 1 to 3 wherein $R_1$ is D-Tyr and $R_{27}$ is Nle, Val or Ile.

10. A process in accordance with Claim 1 for producing a synthetic peptide having the sequence: His - Ala - Asp - Ala - Ile - Phe - Thr - Ser - Ser - Tyr - Arg - Arg - Ile - Leu - Gly - Gln - Leu - Tyr - Ala - Arg - Lys - Leu - Leu - His - Glu - Ile - Met - Asn - Arg - Gln - Gln - Gly - Glu - Arg - Asn - Gln - Glu - Gln - Arg - Ser - Arg - Phe - Asn.

11. A process in accordance with any one of Claims 1—10 wherein residues 33 through 44 are deleted.

12. A method of improving agriculture wherein release of GH is warm-blooded non-human animals is stimulated to promote increase in muscle mass by an effective amount of a peptide or salt as defined in Claim 1.

13. A method of improving aquiculture wherein release of GH in cold-blooded animals is stimulated to accelerate the growth thereof by an effective amount of a peptide or salt as defined in Claim 1.

**Patentansprüche für die Vertragsstaaten: BE, CH, DE, FR, GB, IT, LI, LU, NL, SE**

1. H - $R_1$ - Ala - Asp - Ala - Ile - Phe - Thr - $R_8$ - Ser - $R_{10}$ - Arg - $R_{12}$ - $R_{13}$ - Leu - $R_{15}$ - Gln - Leu - $R_{18}$ - Ala - Arg - Lys - Leu - Leu - $R_{24}$ - $R_{25}$ - Ile - $R_{27}$ - $R_{28}$ - Arg - Gln - Gln - Gly - Glu - $R_{34}$ - Asn - Gln - Glu - $R_{38}$ - $R_{39}$ - $R_{40}$ - Arg - $R_{42}$ - $R_{43}$ - $R_{44}$ - Y worin $R_1$ Tyr, Met, D-Tyr, Phe, D-Phe, Leu, D-His oder His bedeutet, $R_8$ Ser oder Asn bedeutet; $R_{10}$ Tyr, Phe oder D-Tyr bedeutet; $R_{12}$ Arg oder Lys bedeutet; $R_{13}$ Ile oder Val bedeutet; $R_{15}$ Gly oder D-Ala bedeutet; $R_{18}$ Tyr oder Ser bedeutet; $R_{24}$ His oder Gln bedeutet; $R_{25}$ Glu oder Asp bedeutet; $R_{27}$ aus der Gruppe bestehend aus den D- und L-Isomeren von Ala, Nle, Ile, Leu, Met und Val gewählt ist; $R_{28}$ Ser, Asn oder D-Ala bedeutet; $R_{34}$ Arg oder Ser bedeutet; $R_{38}$ Gln oder Arg bedeutet; $R_{39}$ Arg oder Gly bedeutet; $R_{40}$ Ser oder Ala bedeutet; $R_{42}$ Phe oder Ala bedeutet; $R_{43}$ Asn oder Arg bedeutet; $R_{44}$ eine natürliche Aminosäure oder des-$R_{44}$ bedeutet; und Y die Carboxylgruppe des Aminosäurerestes an dem C-Terminus bedeutet und das Radikal —COOR, —CRO, —CONHNHR, —CON(R)(R') oder —$CH_2OR$ ist, wobei R und R' niedriges Alkyl, Fluorniedrigalkyl oder Wasserstoff sind, jedoch mit dem Proviso, daß, wenn $R_1$ Tyr bedeutet, dann bedeutet $R_{27}$ etwas anderes als Met, $R_8$ Ser bedeutet, $R_{12}$ Arg bedeutet, $R_{13}$ Ile bedeutet, $R_{18}$ bedeutet, $R_{24}$ His bedeutet, $R_{25}$ Glu bedeutet, $R_{28}$ Asn bedeutet, $R_{34}$ Arg bedeutet, $R_{38}$ Gln bedeutet, $R_{39}$ Arg bedeutet, $R_{40}$ Ser bedeutet, $R_{42}$ Phe bedeutet oder $R_{43}$ Asn bedeutet, mit dem weiteren Proviso, daß einige oder alle der Reste 28 bis 44 entfernt werden können, um ein biologisch wirksames Fragment oder ein nicht-toxisches Salz desselben, zur Verfügung zum stellen.

2. Ein synthetisches Peptid, welches die Formel von Anspruch 1 besitzt, worin $R_1$ His bedeutet.

3. Ein synthetisches Peptid, welches die Formel von Anspruch 1 oder 2 besitzt, worin Y $CONH_2$ bedeutet.

4. Ein synthetisches Peptid, welches die Formel von einem der Ansprüche 1 bis 3 besitzt, worin $R_{27}$ Nle bedeutet.

5. Ein synthetisches Peptid, welches die Formel von einem der Ansprüche 1 bis 4 besitzt, worin $R_{10}$ Tyr, $R_{15}$ Gly und $R_{28}$ Ser bedeutet.

6. Ein synthetisches Peptid, welches die Formel von einem der Ansprüche 1 bis 4 besitzt, worin $R_{10}$ Tyr, $R_{15}$ D-Ala und $R_{28}$ D-Ala bedeutet.

7. Ein synthetisches Peptid, welches die Formel von Anspruch 1 besitzt, worin $R_1$ D-Tyr bedeutet und worin $R_{27}$ Nle, Val oder Ile bedeutet.

8. Ein synthetisches Peptid, welches entweder die Formel von Anspruch 1 oder von Anspruch 7 besitzt, worin $R_{15}$ D-Ala und $R_{28}$ D-Ala bedeutet.

9. Ein synthetisches Peptid nach Anspruch 1 mit der Sequenz His - Ala - Asp - Ala - Ile - Phe - Thr - Ser - Ser - Tyr - Arg - Arg - Ile - Leu - Gly - Gln - Leu - Tyr - Ala - Arg - Lys - Leu - Leu - His - Glu - Ile - Met - Asn - Arg - Gln - Gln - Gly - Glu - Arg - Asn - Gln - Glu - Gln - arg - Ser - Arg - Phe - Asn.

10. Ein synthetisches Peptid nach einem der Ansprüche 1 bis 9, worin die Reste 33 bis 44 entfernt sind.

11. Ein nicht-toxisches Salz eines Peptids nach einem der Ansprüche 2 bis 10.

12. Eine Zusammensetzung zur Stimulierung der Freisetzung von GH in einem Tier, welche ein Peptid nach einem der Ansprüche 1 bis 10 oder ein nicht-toxisches Salz davon und ein veterinär oder pharmazeutisch verträglicher flüssiger oder fester Träger dafür, enthält.

13. Ein Verfahren zur Verbesserung der Landwirtschaft, mit welchem die Freisetzung von GH in warmblütigen, nicht menschlichen Tieren stimuliert wird, um das Wachstum der Muskelmasse durch eine wirksame Menge eines Peptids oder Salzes nach Anspruch 1 zu fördern.

14. Ein Verfahren zur Verbesserung der Fischzucht, mit welchem die Freisetzung von GH in Kaltbluttieren stimuliert wird, um das Wachstum durch eine wirksame Menge eines Peptids oder eines Salzes nach Anspruch 1 zu beschleunigen.

**Patentansprüche für den Vertragsstaat: AT**

1. Ein Verfahren zur Herstellung von Verbindungen der Formel (I)

$$H - R_1 - Ala - Asp - Ala - Ile - Phe - Thr - R_8 - Ser - R_{10} - Arg - R_{12} - R_{13} - Leu - R_{15} - Gln - Leu - R_{18} - Ala - Arg - Lys - Leu - Leu - R_{24} - R_{25} - Ile - R_{27} - R_{28} - Arg - Gln - Gln - Gly - Glu - R_{34} - Asn - Gln - Glu - R_{38} - R_{39} - R_{40} - Arg - R_{42} - R_{43} - R_{44} - Y$$

worin $R_1$ Tyr, Met, D-Tyr, Phe, D-Phe, Leu, D-His oder His bedeutet, $R_8$ Ser oder Asn bedeutet; $R_{10}$ Tyr, Phe oder D-Tyr bedeutet; $R_{12}$ Arg oder Lys bedeutet; $R_{13}$ Ile oder Val bedeutet; $R_{15}$ Gly oder D-Ala bedeutet; $R_{18}$ Tyr oder Ser bedeutet; $R_{24}$ His oder Gln bedeutet; $R_{25}$ Glu oder Asp bedeutet; $R_{27}$ aus der Gruppe bestehend aus den D- und L-Isomeren von Ala, Nle, Ile, Leu, Met und Val gewählt ist; $R_{28}$ Ser, Asn oder D-Ala bedeutet; $R_{34}$ Arg oder Ser bedeutet; $R_{38}$ Gln oder Arg bedeutet; $R_{39}$ Arg oder Gly bedeutet; $R_{40}$ Ser oder Ala bedeutet; $R_{42}$ Phe oder Ala bedeutet; $R_{43}$ Asn oder Arg bedeutet; $R_{44}$ eine natürliche Aminosäure oder des-$R_{44}$ bedeutet; und Y die Carboxylgruppe des Aminosäurerestes an dem C-Terminus bedeutet und das Radikal —COOR, —CRO, —CONHNHR, —CON(R)(R') oder —CH$_2$OR ist, wobei R und R' niedriges Alkyl, Fluorniedrigalkyl oder Wasserstoff sind, jedoch mit dem Proviso, daß, wenn $R_1$ Tyr bedeutet, dann bedeutet $R_{27}$ etwas anderes als Met, $R_8$ Ser bedeutet, $R_{12}$ Arg bedeutet, $R_{13}$ Ile bedeutet, $R_{18}$ Tyr bedeutet, $R_{24}$ His bedeutet, $R_{25}$ Glu bedeutet, $R_{28}$ Asn bedeutet, $R_{34}$ Arg bedeutet, $R_{38}$ Gln bedeutet, $R_{39}$ Arg bedeutet, $R_{40}$ Ser bedeutet, $R_{42}$ Phe bedeutet oder $R_{43}$ Asn bedeutet, mit dem weiteren Proviso, daß einige oder alle der Reste 28 bis 44 entfernt werden können, um ein biologisch wirksames Fragment oder ein nicht-toxisches Salz desselben, zur Verfügung zu stellen, welches (a) die Bildung eines Peptids mit zumindest einer Schutzgruppe und der Formel (II):

$$X^1 - R_1 (X \text{ oder } X^2) - Ala - Asp(X^3) - Ala - Ile - Phe - Thr(X^4) - R_8(X^4 \text{ oder } X^5) - Ser(X^4) - R_{10}(X^2) - Arg(X^6) - R_{12}(X^6 \text{ oder } X^7) - R_{13} - Leu - R_{15} - Gln(X^5) - Leu - R_{18} - (X^2) - Ala - Arg(X^6) - Lys(X^7) - Leu - Leu - R_{24}(X \text{ oder } X^5) - R_{25}(X^3) - Ile - R_{27} - R_{28}(X^4 \text{ oder } X^5) - Arg(X^6) - Gln(X^5) - Gln(X^5) - Gly - Glu(X^3) - R_{34}(X^4 \text{ oder } X^6) - Asn(X^5) - Gln(X^5) - Glu(X^3) - R_{38}(X^5 \text{ oder } X^6) - R_{39}(X^6) - R_{40}(X^4) - Arg(X^6) - R_{42} - R_{43}(X^5 \text{ oder } X^6) - R_{44}(X^8) - X^9,$$

worin $X$, $X^1$, $X^2$, $X^3$, $X^4$, $X^5$, $X^6$, $X^7$ und $X^8$ entweder jeweils Wasserstoff oder eine Schutzgruppe bedeuten und $X^9$ entweder eine Schutzgruppe oder eine Verankungsbindung an den Harzträger oder des-$X^9$ bedeutet, in welchem Fall der Rest an dem C-Ende eine Y bedeutende Carboxylgruppe besitzt; (b) das Abspalten der Schutzgruppe oder -gruppen oder der Verankerungsbindung von dem Peptid der Formel (II); und (c) gegebenenfalls das Überführen eines erhaltenen Peptids in ein nicht-toxisches Salz davon umfaßt.

2. Ein Verfahren nach Anspruch 1, worin $X^1$ Wasserstoff oder eine α-Amino-Shutzgruppe bedeutet; X Wasserstoff oder eine Schutzgruppe für den Imidazol-Stickstoff von His bedeutet; $X^2$ Wasserstoff oder eine Schutzgruppe für die phenolische Hydroxylgruppe von Tyr bedeutet; $X^3$ Wasserstoff oder eine Schutzgruppe für die Carboxylgruppe von Asp oder Glu bedeutet; $X^4$ Wasserstoff oder eine Schutzgruppe für die alkoholische Hydroxylgruppe von Ser und Thr bedeutet; $X^5$ Wasserstoff oder eine Schutzgruppe für die Amidogruppe von Asn oder Gln bedeutet; $X^6$ Wasserstoff oder eine Schutzgruppe für die Guanidinogruppe von Arg bedeutet; $X^7$ Wasserstoff oder eine Schutzgruppe für die Seitenkettenaminogruppe von Lys bedeutet.

3. Ein Verfahren nach Anspruch 2, worin X Wasserstoff oder Tos ist, $X^1$ BOX ist, $X^2$ DCB ist, $X^3$ OBzl ist, $X^4$ Bzl ist, $X^5$ Xan ist, $X^6$ Tos ist, $X^7$ 2 - Chlorobenzyloxycarbonyl ist und $X^8$ —NH-Harzträger ist.

4. Ein Verfahren nach einem der Ansprüche 1 bis 3, worin $R_1$ His bedeutet.

5. Ein Verfahren nach einem der Ansprüche 1 bis 4, worin Y CONH$_2$ bedeutet.

6. Ein Verfahren nach einem der Ansprüche 1 bis 5, worin $R_{27}$ Nle bedeutet.

7. Ein Verfahren nach einem der Ansprüche 1 bis 6, worin $R_{10}$ Tyr, $R_{15}$ Gly und $R_{28}$ Ser bedeutet.

8. Ein Verfahren nach einem der Ansprüche 1 bis 6, worin $R_{10}$ Tyr, $R_{15}$ D-Ala und $R_{28}$ D-Ala bedeutet.

9. Ein Verfahren nach einem der Anuprüche 1 bis 3, worin $R_1$ D-Tyr bedeutet und $R_{27}$ Nle, Val oder Ile bedeutet.

10. Ein Verfahren nach Anspruch 1 zur Herstellung eines synthetischen Peptids der Sequenz: His - Ala - Asp - Ala - Ile - Phe - Thr - Ser - Ser - Tyr - Arg - Arg - Ile - Leu - Glu - Gln - Leu - Tyr - Ala - Arg - Lys - Leu - Leu - His - Glu - Ile - Met - Asn - Arg - Gln - Gln - Gly - Glu - Arg - Asn - Gln - Glu - Gln - Arg - Ser - Arg - Phe - Asn.

11. Ein Verfahren nach einem der Ansprüche 1 bis 10, worin die Reste 33 bis 44 entfernt sind.

12. Ein Verfahren zur Verbesserung der Landwirtschaft, mit welchem die Freisetzung von GH in warmblütigen, nicht menschlichen Tieren stimuliert wird, um das Wachstum der Muskelmasse durch eine wirksame Menge eines Peptids oder Salzes nach Anspruch 1 zu fördern.

13. Ein Verfahren zur Verbesserung der Fischzucht, mit welchem die Freisetzung von GH in Kaltbluttieren stimuliert wird, um das Wachstum durch eine wirksame Menge eines Peptids oder eines Salzes nach Anspruch 1 zu beschleunigen.

**Revendications pour les Etats Contractants: BE, CH, DE, FR, GB, IT, LI, LU, NL, SE**

1. H - $R_1$ - Ala - Asp - Ala - Ile - Phe - Thr - $R_8$ - Ser - $R_{10}$ - Arg - $R_{12}$ - $R_{13}$ - Leu - $R_{15}$ - Gln - Leu - $R_{18}$ - Ala - Arg - Lys - Leu - Leu - $R_{24}$ - $R_{25}$ - Ile - $R_{27}$ - $R_{28}$ - Arg - Gln - Gln - Gly - Glu - $R_{34}$ - Asn - Gln - Glu - $R_{38}$ - $R_{39}$ - $R_{40}$ - Arg - $R_{42}$ - $R_{43}$ - $R_{44}$ - Y où $R_1$ est Tyr, Met, D-Tyr, Phe, D-Phe, Leu, D-His ou His; $R_8$ est Ser ou Asn; $R_{10}$ est Tyr, Phe ou D-Tyr; $R_{12}$ est Arg ou Lys; $R_{13}$ est Ile ou Val; $R_{15}$ est Gly ou D-Ala; $R_{18}$ est Tyr ou Ser; $R_{24}$ est His ou Gln; $R_{25}$ est Glu ou Asp; $R_{27}$ est choisi dans le groupe comprenant les isomères D et L de Ala, Nle, Ile, Leu, Met et Val; $R_{28}$ est Ser, Asn ou D-Ala; $R_{34}$ est Arg ou Ser; $R_{38}$ est Gln ou Arg; $R_{39}$ est Arg ou Gly; $R_{40}$ est Ser ou Ala; $R_{42}$ est Phe ou Ala; $R_{43}$ est Asn ou Arg; $R_{44}$ est un acide aminé naturel ou des-$R_{44}$; et Y représente le fragment carboxyle du résidu d'acide aminé se trouvant sur l'atome de carbone terminal, et est le radical —COOR, —CRO, —CONHNHR, —CON(R)(R') ou —$CH_2OR$, R et R' étant chacun un radical alkyle inférieur, fluoro(alkyle inférieur) ou hydrogène, à la condition toutefois que, quand $R_1$ est Tyr, $R_{27}$ soit autre que Met, $R_8$ soit Ser, $R_{12}$ soit Arg, $R_{13}$ soit Ile, $R_{18}$ soit Tyr, $R_{24}$ soit His, $R_{25}$ soit Glu, $R_{28}$ soit Asn, $R_{34}$ soit Arg, $R_{38}$ soit Gln, $R_{39}$ soit Arg, $R_{40}$ soit Ser, $R_{42}$ soit Phe ou $R_{43}$ soit Asn; à la condition supplémentaire que tout ou partie des résidus 28 à 44 puissent être supprimés pour fournir un fragment biologiquement actif, ou un sel non toxique des composés ci-dessus.

2. Peptide synthétique ayant la formule de la revendication 1, où $R_1$ est His.

3. Peptide synthétique ayant la formule de l'une quelconque des revendications 1 ou 2, où Y est $CONH_2$.

4. Peptide synthétique ayant la formule de l'une quelconque des revendications 1—3, où $R_{27}$ est Nle.

5. Peptide synthétique ayant la formule de l'une quelconque des revendications 1 à 4, où $R_{10}$ est Tyr, $R_{15}$ est Gly et $R_{28}$ est Ser.

6. Peptide synthétique ayant la formule de l'une quelconque des revendications 1 à 4, où $R_{10}$ est Tyr, et $R_{15}$ est D-Ala et $R_{28}$ est D-Ala.

7. Peptide synthétique ayant la formule de la revendication 1, où $R_1$ est D-Tyr et où $R_{27}$ est Nle, Val ou Ile.

8. Peptide synthétique ayant la formule de l'une quelconque des revendications 1 ou 7, où $R_{15}$ est D-Ala et $R_{28}$ est D-Ala.

9. Peptide synthétique selon la revendication 1, ayant la séquence His - Ala - Asp - Ala - Ile - Phe - Thr - Ser - Ser - Tyr - Arg - Arg - Ile - Leu - Gly - Gln - Leu - Tyr - Ala - Arg - Lys - Leu - Leu - His - Glu - Ile - Met - Asn - Arg - Gln - Gln - Gly - Glu - Arg - Asn - Gln - Glu - Gln - Arg - Ser - Arg - Phe - Asn.

10. Peptide synthétique selon l'une quelconque des revendications 1—9, dans lequel les résidus 33 à 44 sont supprimés.

11. Sel non-toxique d'un peptide selon l'une quelconque des revendications 2 à 10.

12. Composition pour stimuler la libération de l'hormone somatotrope GH chez l'animal, laquelle composition comprend un peptide selon l'une quelconque des revendications 1 à 10 ou l'un de ses sels non-toxiques, et un excipient liquide ou solide, acceptable d'un point de vue vétérinaire ou pharmaceutique, pour cette composition.

13. Procédé de perfectionnement agricole, dans lequel la libération de l'hormone somatotrope GH chez des animaux non-humains à sang chaud est stimulée de façon à favoriser une augmentation de la masse musculaire, sous l'effet d'une quantité efficace d'un peptide ou d'un sel tels que définis dans la revendication 1.

14. Procédé de perfectionnement aquicole, dans lequel la libération de l'hormone somatotrope GH chez des animaux à sang froid est stimulée de façon à accélérer leur croissance, sous l'effet d'une quantité efficace d'un peptide ou d'un sel tels que définis dans la revendication 1.

**Revendications pour l'Etat Contractant: AT**

1. Procédé de préparation de composés définis par la formule (I):

H - $R_1$ - Ala - Asp - Ala - Ile - Phe - Thr - $R_8$ - Ser - $R_{10}$ - Arg - $R_{12}$ - $R_{13}$ - Leu - $R_{15}$ - Gln - Leu - $R_{18}$ - Ala - Arg - Lys - Leu - Leu - $R_{24}$ - $R_{25}$ - Ile - $R_{27}$ - $R_{28}$ - Arg - Gln - Gln - Gly - Glu - $R_{34}$ - Asn - Gln - Glu - $R_{38}$ - $R_{39}$ - $R_{40}$ - Arg - $R_{42}$ - $R_{43}$ - $R_{44}$ - Y,

où $R_1$ est Tyr, Met, D-Tyr, Phe, D-Phe, Leu, D-His ou His; $R_8$ est Ser ou Asn; $R_{10}$ est Tyr, Phe ou D-Tyr; $R_{12}$ est Arg ou Lys; $R_{13}$ est Ile ou Val; $R_{15}$ est Gly ou D-Ala; $R_{18}$ est Tyr ou Ser; $R_{24}$ est His ou Gln; $R_{25}$ est Glu ou Asp; $R_{27}$ est choisi dans le groupe comprenant les isomères D et L de Ala, Nle, Ile, Leu, Met et Val; $R_{28}$ est Ser, Asn ou D-Ala; $R_{34}$ est Arg ou Ser; $R_{38}$ est Gln ou Arg; $R_{39}$ est Arg ou Gly; $R_{40}$ est Ser ou Ala; $R_{42}$ est Phe ou Ala; $R_{43}$ est Asn ou Arg; $R_{44}$ est un acide aminé naturel ou des-$R_{44}$; et Y représente le fragment carboxyle du résidu d'acide aminé se trouvant sur l'atome de carbone terminal, et est le radical —COOR, —CRO, —CONHNHR, —CON(R)(R') ou —$CH_2OR$, R et R' étant chacun un radical alkyl inférieur, fluoro(alkyle inférieur) ou hydrogène, à la condition toutefois que, quand $R_1$ est Tyr, $R_{27}$ soit autre que Met, $R_8$ soit Ser, $R_{12}$ soit Arg, $R_{13}$ soit Ile, $R_{18}$ soit Tyr, $R_{24}$ soit His, $R_{25}$ soit Glu, $R_{28}$ soit Asn, $R_{34}$ soit Arg, $R_{38}$ soit Gln, $R_{39}$ soit Arg, $R_{40}$ soit Ser, $R_{42}$ soit Phe ou $R_{43}$ soit Asn; à la condition supplémentaire que tout ou partie des résidus 28 à 44 puissent être supprimés pour fournir un fragment biologiquement actif, ou un sel non toxique des composés ci-dessus; consistant (a) à former un peptide ayant au moins un groupe protecteur et la formule (II):

$$X^1 - R_1(X \text{ ou } X^2) - Ala - Asp(X^3) - Ala - Ile - Phe - Thr(X^4) - R_8(X^4 \text{ ou } X^5) - Ser(X^4) - R_{10}(X^2) -$$
$$Arg(X^6) - R_{12}(X^6 \text{ ou } X^7) - R_{13} - Leu - R_{15} - Gln(X^5) - Leu - R_{18}(X^2) - Ala - Arg(X^6) - Lys(X^7) -$$
$$Leu - Leu - R_{24}(X \text{ ou } X^5) - R_{25}(X^3) - Ile - R_{27} - R_{28}(X^4 \text{ ou } X^5) - Arg(X^6) - Gln(X^5) - Gln(X^5) - Gly -$$
$$Glu(X^3) - R_{34}(X^4 \text{ ou } X^6) - Asn(X^5) - Gln(X^5) - Glu(X^3) - R_{38}(X^5 \text{ ou } X^6) - R_{39}(X^6) - R_{40}(X^4) -$$
$$Arg(X^6) - R_{42} - R_{43}(X^5 \text{ ou } X^6) - R_{44}(X^8) - X^9$$

dans laquelle X, $X^1$, $X^2$, $X^3$, $X^4$, $X^5$, $X^6$, $X^7$ et $X^8$ sont chacun un hydrogène ou un groupe protecteur, et $X^9$ est un groupe protecteur ou une liaison de fixation à un support résine ou des-$X^9$, auquel cas le résidu, sur l'atome de carbone terminal, possède un fragment carboxy qui est Y; (b) à éliminer le ou les groupes protecteurs ou la liaison de fixation dudit peptide de formule (II); et (c), si on le souhaite, à convertir le peptide obtenu en l'un de ses sels non-toxiques.

2. Procédé selon la revendication 1, dans lequel $X^1$ est un hydrogène ou un groupe α-amino-protecteur; X est un hydrogène ou un groupe protecteur de l'azote du noyau imidazole de His; $X^2$ est un hydrogène ou un groupe protecteur du groupe hydroxyle phénolique de Tyr; $X^3$ est un hydrogène ou un groupe protecteur du groupe carboxyle de Asp ou Glu; $X^4$ est un hydrogène ou un groupe protecteur du groupe hydroxyle alcoolique de Ser et Thr; $X^5$ est un hydrogène ou un groupe protecteur du groupe amido de Asn ou Gln; $X^6$ est un hydrogène ou un groupe protecteur du groupe guanidino de Arg; $X^7$ est un hydrogène ou un groupe protecteur du groupe amino en chaîne latérale de Lys.

3. Procédé selon la revendication 2, dans lequel X est un hydrogène ou Tos, $X^1$ est BOC, $X^2$ est DCB, $X^3$ est OBzl, $X^4$ est Bzl, $X^5$ est Xan, $X^6$ est Tos, $X^7$ est le radical 2 - chlorobenzyloxycarbonyle et $X^8$ est —NH-support résine.

4. Procédé selon l'une quelconque des revendications 1—3, dans lequel $R_1$ est His.

5. Procédé selon l'une quelconque des revendications 1—4, dans leuqel Y est $CONH_2$.

6. Procédé selon l'une quelconque des revendications 1—5, dans lequel $R_{27}$ est Nle.

7. Procédé selon l'une quelconque des revendications 1—6, dans lequel $R_{10}$ est Tyr, $R_{15}$ est Gly et $R_{28}$ est Ser.

8. Procédé selon l'une quelconque des revendications 1 à 6, dans lequel $R_{10}$ est Tyr, $R_{15}$ est D-Ala et $R_{28}$ est D-Ala.

9. Procédé selon l'une quelconque des revendications 1 à 3, dans lequel $R_1$ est D-Tyr et $R_{27}$ est Nle, Val ou Ile.

10. Procédé selon la revendication 1, pour préparer un peptide synthétique selon la revendication 1, ayant la séquence His - Ala - Asp - Ala - Ile - Phe - Thr - Ser - Ser - Tyr - Arg - Arg - Ile - Leu - Gly - Gln - Leu - Tyr - Ala - Arg - Lys - Leu - Leu - His - Glu - Ile - Met - Asn - Arg - Gln - Gln - Gly - Glu - Arg - Asn - Gln - Glu - Gln - Arg - Ser - Arg - Phe - Asn.

11. Procédé selon l'une quelconque des revendications 1—10, dans lequel les résidus 33 à 44 sont supprimés.

12. Procédé de perfectionnement agricole, dans lequel la libération de l'hormone somatotrope GH chez des animaux non-humains à sang chaud est stimulée de façon à favoriser une augmentation de la masse musculaire, sous l'effet d'une quantité efficace d'un peptide ou d'un sel tels que définis dans la revendication 1.

13. Procédé de perfectionnement aquicole, dans lequel la libération de l'hormone somatotrope GH chez des animaux à sang froid est stimulée de façon à accélérer leur croissance, sous l'effet d'une quantité efficace d'un peptide ou d'un sel tels que définis dans la revendication 1.